# EUROPÄISCHE PATENTSCHRIFT

(11) **EP 1 299 039 B1**
(45) Veröffentlichungstag und Bekanntmachung des Hinweises auf die Patenterteilung: **29.11.2006**
(21) Anmeldenummer: 01957705.5
(22) Anmeldetag: 05.07.2001
(51) Int. Cl.: A61B 17/32, A61P 35/00, A61P 41/00, A61K 9/08

(54) **HOCHDRUCKFLÜSSIGKEITSSTRAHLEINRICHTUNG MIT EINEM FLÜSSIGEN MEDIKAMENT**
SURGICAL HIGH PRESSURE LIQUID JET DEVICE WITH LIQUID MEDICAMENT
DISPOSITIF CHIRURGICAL A JET DE LIQUIDE HAUTE PRESSION AVEC MEDICAMENT LIQUIDE

(30) Priorität: 07.07.2000 DE 10033283
(43) Veröffentlichungstag der Anmeldung: 09.04.2003
(73) Patentinhaber: Human Med AG, 19061 Schwerin (DE)
(72) Erfinder: KÖCKERLING, Ferdinand, 30177 Hannover (DE)
(74) Vertreter: Jaap, Reinhard
(86) Internationale Anmeldenummer: PCT/DE2001/002416
(87) Internationale Veröffentlichungsnummer: WO 2002/003871

(56) Entgegenhaltungen:
- WO-A-99/66848
- US-A- 4 560 373
- DATABASE WPI Week 20036 Derwent Publications Ltd., London, GB; AN 2000-420834 XP002181523 & RU 2 136 221 C (GLUKHOV), 10. September 1999 (1999-09-10)
- DATABASE WPI Week 9813 Derwent Publications Ltd., London, GB; AN 1998-143661 XP002181524 & RU 2 085 200 C (ROST ONCOLOGY RES INST), 27. Juli 1997 (1997-07-27)
- DATABASE BIOSIS [Online] BIOSCIENCES INFORMATION SERVICE, PHILADELPHIA, PA, US; prev199800168788, P. JACQUET ET AL.: "Heated intraoperative intraperitoneal mitomycin C and early postoperative intraperitoneal 5-fluorouracil: pharmacokinetic studies" XP002181522 & ONCOLOGY, Bd. 55, Nr. 2, März 1998 (1998-03), Seiten 130-138, Basel (CH)

## Beschreibung

Gegenstand der Erfindung ist eine Hochdruckflüssigkeitsstrahleinrichtung mit einer zytotoxischen Flüssigkeit zur Bekämpfung von Tumoren bzw. Tumorzellen zur Behandlung von Dissektionsebenen / Resektionsflächen an einer biologischen Struktur und zur gleichzeitigen Trennung einer biologischen Struktur nach Anspruch 1.
Derartige Hochdruckflüssigkeitsstrahleinrichtungen mit den entsprechenden flüssigen Medikamenten werden in chirurgischen Kliniken eingesetzt.

Zytotoxische Flüssigkeiten sind beispielsweise aus der XP-002181522 bekannt.

Dissektionsebenen / Resektionsflächen der allgemeinen Art werden zunächst operativ erzeugt und anschließend medikamentös behandelt, wobei die medikamentöse Behandlung sich auf ein Betupfen, ein Überstreichen oder ein Übergießen der Dissektionsebenen / Resektionsflächen mit einem Medikament beschränkt. Beide Arbeitsschritte liegen demnach zeitlich auseinander, was den zusammenhängenden Operationsprozess verlängert und den Behandlungserfolg beeinträchtigt. Ein weiterer Nachteil besteht darin, dass die nachfolgende Behandlung der Dissektionsebenen / Resektionsflächen nur oberflächlich erfolgt und die Wirkstoffe des Medikamentes nur sehr langsam in das Gewebe der biologischen Struktur eindringen kann. Das verlängert den Heilungsprozess.
Dissektionsebenen / Resektionsflächen der besonderen Art entstehen beim operativen Eingriff zur Beseitigung von Tumoren, die dann auch in besonderer Weise behandelt werden müssen. Tumore sind gutartige oder bösartige Geschwülste, die sich in die verschiedensten Organe mit den umgebenden Lymph-, Fett-, und Bindegewebe und der Muskulatur oder in einer anderen biologischen Struktur einbetten. Die bösartigen Tumore wachsen sehr schnell und dringen dabei über die Lymphbahnen und die Lymphspalte in das benachbarte und gesunde Gewebe ein, wo sie dann Metastasen ausbilden.
Solche Tumore werden in der Regel operativ behandelt, in dem das jeweilige tumortragende Organ oder eine andere tumortragende biologische Struktur aus dem Tumorbett ausgetrennt wird. Dabei ist trotz einer oftmals radikalen Erweiterung des Sicherheitsabstandes nicht zu verhindern, dass vitale Tumorzellen in den Lymphbahnen und Lymphspalten des Tumorbettgewebes vom Operationsschnitt nicht erfasst werden, die dann im Tumorbettgewebe verbleiben oder auch während der Operation aus dem ehemaligen Tumorbett austreten. Daher wird nach der Entfernung des tumortragenden Organs oder der anderen tumortragenden biologischen Struktur das bisherige Tumorbett mit einer zytotoxischen Flüssigkeit ausgespült, um die im Tumorbett befindlichen und die im Tumorbettgewebe verbliebenen Tumorzellen abzutöten. Mit diesem Spülverfahren werden die Wirkstoffe der zytotoxischen Flüssigkeit jedoch nur sehr ungenau und unpräzise an die Orte des Tumorgeschehens herangebracht, so dass nur oberflächliche Tumorzellen erreicht werden und ein erheblicher Anteil von Tumorzellen im Tumorbett oder im Tumorbettgewebe unbehandelt bleiben. Das Risiko eines Tumorrückfalles ist daher sehr groß, wie die ermittelte Rückfallrate beweist.
Daran können auch bestimmte Nachfolgemaßnahmen wie beispielsweise eine systematische Chemotherapie oder eine Bestrahlung nichts ändern.

Der Erfindung liegt daher die Aufgabe zu Grunde, eine Vorrichtung zu entwickeln, die auch tieferliegende Bereiche der Dissektionsebenen / Resektionsflächen an einer biologischen Struktur erreicht.

Diese Aufgabe wird durch die kennzeichnenden Merkmale des Anspruches 1 gelöst.

Mit der Erfindung werden die genannten Nachteile aus dem Stand der Technik beseitigt. Dabei liegt der besondere Vorteil der Erfindung darin, dass zwei eigentlich gesonderte Operationsvorgänge miteinander verknüpft und kombiniert werden.
So wird zum einen das flüssige Medikament in erfinderischer Weise zur Trennung der biologischen Struktur eingesetzt und dabei die gleiche Wirkung wie mit einem herkömmlichen sterilen Trennmittel erzielt. Mit dem Einsatz des Medikamentes als Trennmedium beginnt das Medikament bereits während, also in zeitlicher Überlagerung mit dem Trennvorgang zu wirken. Das verkürzt einerseits den Operationsprozess und intensiviert andererseits den Behandlungsprozess.
So wird zum anderen das flüssige Medikament in einer neuartigen Applikationsart angewendet, in dem es nicht mehr durch Überspülen der Dissektionsebenen / Resektionsflächen von außen aufgetragen, sondern in injizierender Weise in das angrenzende Gewebebett eingegeben wird. Das ergibt eine neue Wirkung, denn erstmals können damit auch tieferliegende z.B. Bakterien oder Tumorzellen erreicht und behandelt werden.
Grundlage der vorteilhaften Operations- und Behandlungsmethode ist eine Hochdruckflüssigkeitseinrichtung, die in der Lage ist, einen feinen und trennfähigen Flüssigkeitsstrahl zu erzeugen. Eine solche Einrichtung verfügt auch über eine stufenlos veränderbare Druckregeleinrichtung, die eine feinfühlige Flüssigkeitsstrahlregulierung erlaubt. Damit ist es im vorliegenden Operationsverfahren möglich, die Tiefenwirkung des Medikamentenstrahles auf die speziellen Anforderungen des Krankheitsfalles anzupassen.
Alle diese positiven Wirkungen führen dazu, dass alle Bakterien oder von einer Krankheit befallenden Gewebezellen optimal und zwar an ihrem Aufenthaltsort behandelt und bekämpft werden und damit ein Rückfall der Krankheit ausgeschlossen ist.

Die Erfindung soll anhand eines Ausführungsbeispieles näher erläutert werden.

Zur Durchführung eines operativen Eingriffes zum Beispiel zur Entfernung eines Tumores wird eine Hochdruckflüssigkeitsstrahleinrichtung eingesetzt, wie sie beispielsweise in der EP 0 551 920 B 1 beschrieben und unter der Handelsbezeichnung HELIX HYDRO-Jet bekannt ist. Diese Hochdruckflüssigkeitsstrahleinrichtung besteht im wesentlichen aus einem Druckerzeuger, einer Kolben-Zylinder-Einheit und einer Trenneinrichtung in Form eines speziellen Handstückes. Im Zylinderraum der Kolben-Zylinder-Einheit ist formschlüssig eine Kartusche eingesetzt, die mit einer Trennflüssigkeit gefüllt ist und die mit dem Operationshandstück verbunden ist. Im Betrieb belastet das Druckmedium des Druckerzeugers die Kartusche, wodurch die Trennflüssigkeit aus der Kartusche gedrückt und zum Operationshandstück gefördert wird, wo sie unter Druck in Form eines feinen Flüssigkeitsstrahles austritt. Alle Geräteeinheiten dieser Hochdruckflüssigkeitsstrahleinrichtung sind so ausgeführt und aufeinander abgestimmt, dass einerseits eine durchgehende Sterilkette für die Trennflüssigkeit gewährleistet ist und andererseits der austretende Strahl der Flüssigkeit in intelligenter Weise unterschiedliche biologische Strukturen in höchster Präzision zu trennen vermag.

Erfindungsgemäß wird als Trennflüssigkeit eine zytotoxische Flüssigkeit verwendet, wie sie allgemein bekannt ist und die druckbeständig bis 150 bar und die in ihrer Viskosität strahlfähig ist.

Im Zuge der Operation wird das tumorbefallende Organ oder die andere biologische Struktur mit der Trennkraft des Strahles der zytotoxischen Flüssigkeit von dem umgebenden Lymph-, Fett- und Bindegewebe sowie von der Muskulatur getrennt. Das geschieht in einem solchen Sicherheitsabstand zum Tumor, dass möglichst viele Metastasen mit abgetrennt werden. Dabei werden der eingebettete Tumor und alle mit dem Trennvorgang aus den Lymphbahnen und den Lymphspalten ins Tumorbett ausgetretenden Metastasen bereits während des Trennvorganges zwangsläufig mit der zytotoxischen Trennflüssigkeit kontaktiert, sodass die betreffenden Tumorzellen zum Absterben veranlasst werden. Gleichzeitig dringt die zytotoxische Flüssigkeit, verursacht durch den hohen Druck des Trennstrahles, tief in die Lymphbahnen und Lymphspalte des ehemaligen Tumorbettgewebes ein und kontaktiert auch die dort befindlichen Metastasen. Somit sterben auch diese betreffenden Tumorzellen ab.

Diese mit dem Trennvorgang ablaufende Behandlung der Tumorzellen ist als eine intestitielle Chemotherapie zu bezeichnen, bei der die zytotoxischen Wirkstoffe der Flüssigkeit mit Hilfe der Hochdruckflüssigkeitsstrahleinrichtung in einer neuen Applikationsform tief in das Tumorbettgewebe eingebracht werden.

## Patentansprüche

1. Hochdruckflüssigkeitsstrahleleinchtung zur Trennung einer biologischen Struktur und zur gleichzeitigen Behandlung von Dissektionsebenen / Resektionsflächen an einer biologischen Struktur, bestehend aus einem Druckerzeuger, einem vom Druckerzeuger unter Druck zu setzenden Vorratsbehälter mit einem Trennmedium und einem Operationshandstück, aus dem das Trennmedium strahlfömig austritt, wobei das Trennmedium ein flüssiges, druckbeständiges und strahlfähiges Medikament ist, **dadurch gekennzeichnet, dass** das flüssige Medikament eine zytotoxische Flüssigkeit zur Bekämpfung von Tumoren ist.

## Claims

1. High-pressure liquid jet device for separating a biological structure and simultaneously treating dissection planes/resection surfaces on a biological structure, comprising a pressure generator, a supply container containing a separating medium to be placed under pressure by the pressure generator and an operating hand-piece from which the separating medium is discharged in a jet, which separating medium is a liquid medicament capable of withstanding pressure and suitable for forming a jet, **characterised in that** the liquid medicament is a cytotoxic liquid for combating tumours.

## Revendications

1. Dispositif à jet de liquide haute pression pour la séparation d'une structure biologique et pour le traitement simultané de plans de dissection/faces de résection sur une structure biologique, se composant d'un générateur de pression, d'un réservoir de stockage à mettre sous pression par le générateur de pression, avec un fluide de séparation et une poignée de manipulation, par laquelle le fluide de séparation sort sous la forme d'un jet, le fluide de séparation étant un médicament liquide, résistant à la pression et pouvant former un jet,
**caractérisé en ce que**
le médicament liquide est un liquide cytotoxique destiné à combattre les tumeurs.
